# EUROPEAN PATENT SPECIFICATION

(11) **EP 3 251 602 B1**
(45) Date of publication and mention of the grant of the patent: **11.09.2024**
(21) Application number: 16172366.3
(22) Date of filing: 01.06.2016
(51) Int. Cl.: A61B 8/00, A61B 8/08, G06T 17/00, G06T 19/20, G16H 50/30

(54) **METHOD FOR ANALYSING A NON-PLANAR ORGAN**
VERFAHREN ZUR ANALYSE EINES UNEBENEN ORGANS
PROCÉDÉ POUR ANALYSER UN ORGANISME NON PLAN

(43) Date of publication of application: 06.12.2017
(73) Proprietor: TomTec Imaging Systems GmbH, 85716 Unterschleissheim (DE)
(72) Inventor: Schummers, Georg, 80686 Munich (DE); Schreckenberg, Marcus, 85356 Freising (DE); Mumm, Bernhard, 82291 Mammendorf (DE)
(74) Representative: Philips Intellectual Property & Standards

(56) References cited:
- US-A1- 2008 167 581
- A. YOUSSEF ET AL: "Reliability of new three-dimensional ultrasound technique for pelvic hiatal area measurement", ULTRASOUND IN OBSTETRICS AND GYNECOLOGY, vol. 47, no. 5, 29 March 2016 (2016-03-29), GB, pages 629 - 635, XP055322770, ISSN: 0960-7692, DOI: 10.1002/uog.14933
- YOUSSEF ET.AL.: "How to assess the pelvic hiatal area by the Omniview-VCI technique", 29 March 2016 (2016-03-29), pages 1 pp., XP054976939, Retrieved from the Internet <URL:http://onlinelibrary.wiley.com/store/10.1002/uog.14933/asset/supinfo/uog14933-sup-0001-VideoS1.mp4?v=1&s=a0a595ad80cbe2a67f0c17b10ac02905d34ed3bf> [retrieved on 20161125]
- K. VAN DELFT ET AL: "Pelvic floor muscle contractility: digital assessment vs transperineal ultrasound", ULTRASOUND IN OBSTETRICS AND GYNECOLOGY, vol. 45, no. 2, 27 January 2015 (2015-01-27), GB, pages 217 - 222, XP055322784, ISSN: 0960-7692, DOI: 10.1002/uog.13456
- J. A. KRUGER ET AL: "How best to measure the levator hiatus: evidence for the non-Euclidean nature of the 'plane of minimal dimensions'", ULTRASOUND IN OBSTETRICS AND GYNECOLOGY, vol. 36, no. 6, 19 July 2010 (2010-07-19), GB, pages 755 - 758, XP055322776, ISSN: 0960-7692, DOI: 10.1002/uog.7750

## Description

The present invention refers to a method for analysing a non-planar organ in the human or animal body, in particular a strand-shaped organ such as the puborectalis muscle, to an appropriate computer program and to a device for analysing a non-planar organ.

During vaginal delivery, the pelvic floor muscles are exposed to extreme stretching. Especially affected is the puborectalis muscle which is a sling- or strand-shaped muscle, starting in two fascicles from the lower pubic symphysis, connecting behind the rectum. When the baby tries to pass through it, the muscle is stretched by 200 up to 1000 % of its normal length. Tearing of this muscle is therefore not uncommon and may cause serious pelvic floor dysfunction which results in incontinence and even organ prolapse. Although some women prepare for vaginal delivery by pelvic floor muscle training to increase muscle elasticity, 10 to 30 % suffer heavy levator trauma like avulsion from the pubic bone or tearing and even more women sustain irreversible muscle over-distension.

The examination of this muscle before delivery may help to obtain information about the muscle's condition and risks of taking damage can be prevented by performing a caesarean section in critical or uncertain cases.

Since Computed Tomography (CT) and Magnetic Resonance Imaging (MRI) are only performed on pregnant women in exceptional cases, ultrasound is the safest way to allow this non-invasive inspection. A recent innovation in ultrasound examinations in this area are 3D (three-dimensional) and 4D (four-dimensional) ultrasound systems which enable real-time imaging, and also allow multi-planar reconstructions (MPRs), which used to be the advantage of MRI. Nevertheless, organs have no exact geometric form or shape and therefore analysis of such 3D datasets is very difficult. By way of example, it is very complex to measure exactly the length of a strand-shaped organ like the puborectalis muscle.

In the article "Reliability of new three-dimensional ultrasound technique for pelvic hiatal area measurement", Ultrasound in Obstetrics and Gynecology, vol. 47, no. 5; 29 March 2016, Youssef et al discloses an OmniView^{™} volume contrast imaging (VCI) for measurement of the pelvic hiatal area on maximum contraction.

### SUMMARY OF THE INVENTION

It is an object of the present invention to provide a method, a computer program and a device which overcome the aforementioned problems and which provide a reliable basis for the analysis of a non-planar organ in the human or animal body.

This object is achieved by a method according to claim 1, by a computer program according to claim 10 and by a device according to claim 11. Further features and advantages are defined in the dependent claims, the description and the figures.

### SUMMARY OF THE DISCLOSURE

According to the disclosure, a method for analysing, in particular measuring, a non-planar organ in the human or animal body, in particular a strand-shaped organ, comprises the steps:
a. providing at least one 3D or 4D image dataset which includes the organ;
b. selecting a basic plane through the 3D image dataset which at least partially intersects the organ;
c. displaying the basic plane and defining the organ on the basic plane, resulting in a model of the organ;
d. providing a curved profile plane (30) which follows the model and extends perpendicular to the basic plane;
e. displaying the curved profile plane and defining the organ on the curved profile plane, resulting in an adjusted model of the organ.

According to one embodiment, a 3D or 4D ultrasound system is used to acquire or generate the 3D or 4D image dataset, wherein the 4^{th} dimension is time. Accordingly, the 3D image dataset defines a 3D data volume. Expediently, a 3D or 4D transperineal ultrasound assessment is carried out to acquire the 3D/4D image dataset, preferably from the pelvic floor muscle area of a woman, in particular of a pregnant woman. The 3D or 4D image dataset may accordingly be acquired in an additional step prior to step a. The inventive analysis method can be performed either directly after the acquisition and by means of the ultrasound system, or at a later time at which the 3D or 4D image datasets may be analysed on a separate computer or other computing device, such as a tablet, laptop etc.

Preferably, the method is performed using a graphical user interface. The graphical user interface preferably comprises viewing areas for displaying image planes which have a user-selected orientation/angle through the 3D image dataset (Multi-planar reconstruction). The graphical user interface also comprises user input devices, such as buttons, sliders or a mouse, touch screen etc.. The user input devices may be used to define the organ on a displayed image plane, for example by selecting points/landmarks or drawing lines onto the displayed image. In addition, the graphical user interface may comprise sliders to shift the image planes in a respective dimension, which enables different views on the organ. The dataset is for example initially displayed on three orthogonal planes, showing slices of a data cube XY, YZ and XZ.

The organ is the puborectalis muscle. It has been shown that the puborectalis muscle is not a planar sling from the pubic symphysis around the rectum but follows a three-dimensionally curved shape. In the prior art, measurements of this muscle structure are made on planar slices and due to its three-dimensional character this holds non-negligible inaccuracy.

Expediently, in a first step a basic plane is selected, which means that a plane has to be found which basically corresponds to a "plane" of the organ to be examined. Of course, the organ to be examined is not plane. Nevertheless, it is possible to define a plane which at least partially intersects the organ which means that the plane lies (at least partially) preferably "in/within" the organ. The "basic plane" is usually a plane that is easily found with the 3D image dataset. According to one embodiment, for example two characteristic points of the organ or characteristic points near the organ can be used to define a basic axis which can be used to define the basic plane. The basic plane can preferably be tilted around the basic axis to adjust the basic plane with regard to the organ.

Preferably, the basic plane is the "plane of minimal dimensions" of the puborectalis muscle according to Dietz (see H.P. Dietz, "Pelvic floor trauma in childbirth", Australian and New Zealand Journal of Obstetrics and Gynaecology 2013; 53: 220-230). The plane of minimal dimensions describes a tilted axial plane cutting through the pelvic floor capturing the levator hiatus at its smallest extent. The plane is defined by a point on the pubic symphysis and posteriorly by the anorectal angle. The method to define the plane of minimal dimensions may be performed as follows:
At first, a landmark for the pubic symphysis is set. This point is visible in an axial plane where a bone structure appears anteriorly. The bones have to be as close together as possible but still clearly separable. Preferably, with a respective slider of the graphical user interface a descriptive plane can be found. The pubic symphysis can also be identified in the coronal plane. Expediently, this spot is saved by clicking for example on the axis of the best view. When the pubic symphysis is marked, the posterior border of the levator hiatus is the subject to find. The anorectal angle is visible in the mid-sagittal view. A bright structure posterior to the anorectal angle is the cross-section of the puborectalis muscle. On the border of this structure as close to the symphysis landmark as possible, the puborectalis muscle landmark has to be set. With these two (characteristic) points the basic plane is defined.

The basic plane with its settings for its orientation angle and position can be defined by a rotation matrix which is also needed to back-project points/landmarks. The rotation matrix preferably comprises three orthogonal vectors describing the plane's unit vectors, and an origin point, defining the plane's position.

According to step c, the organ is defined on the basic plane, in most embodiments this is done by a user selecting a number of e.g. 3 to 10 points or landmarks which fall within the organ. In other words and according to the invention, the user is sketching the shape of the organ, for example by drawing an outline of the organ which is immediately displayed on the basic plane. The sketching may outline the centreline of the organ, or alternatively the external border of the organ. This definition is used as the basis for a (usually very basic) model of the organ, for example a curved line spanned by the points selected by the user on the basic plane. I.e. a simple model of the organ may be produced by the user by selecting only a few points (e.g. 3 to 10) within the organ on the displayed plane, in this case the basic plane. A line, e.g. a spline function, may then be fitted through these points to produce a model, which is planar since the user has only selected points within one plane, the basic plane. In other embodiments, the user may draw a line or more complex shape on the basic plane. In further embodiments, the organ may be defined by the computer automatically, using machine learning methods.

In a next step d, this line-shaped model is used to provide a curved profile plane which follows the curvature of the line and which extends perpendicular to the basic plane. The curved profile plane is generated by first mathematically defining a plane perpendicular to the basic plane, which follows the model sketched on the basic plane. Then, the nearest voxels of the 3D dataset to such curved profile plane are identified and a viewing plane is generated e.g. by interpolating from the nearest voxels in the 3D image dataset.

This viewing plane is then displayed for the user as the curved profile plane. Ideally, the organ will intersect the curved profile plane more than it does the basic plane, but it will not be visible as a straight line. Accordingly, the organ is defined again on the displayed curved profile plane by the same method as before, e.g. selecting a number of points and fitting a spline through the points, or drawing the centreline. This can be done by a user, or automatically. Thus, by sketching the organ on the curved profile plane, an adjusted model is generated. The adjusted model does not necessarily lie in one plane, but extends three-dimensionally.

Preferably, the method comprises the further step:
f. fitting a target plane to the adjusted model or a few landmarks defining the adjusted model; and
g. displaying the target plane and projecting the adjusted model onto the target plane for review by a user;
h. optionally, further amending the model to fit it to the organ on the target plane, resulting in an improved model of the organ.

The target plane may, but need not necessarily be fitted to the whole adjusted model. As is generally known, three points/landmarks are enough to define a plane. Accordingly, in case the organ is the puborectalis muscle, often the two end points and the centre points of the sling (called "the three basic landmarks" in the following) are used to calculate the orientation of a planar target plane.

Then, the adjusted model is projected onto the target plane, and reviewed by the user. If necessary, the model can be amended to improve the fit to the organ, for example by slightly changing the position of one or several selected points or landmarks. The thus improved model of the organ can be used to make measurements of the organ, for example measure its length or other dimensions. Further measurements can be carried out on the displayed target plane. In some embodiments, the projection of the adjusted or improved model onto the target plane is compared to a projection of the original model. The difference between these two projections shows the accuracy of the basic plane.

In some embodiments, the target plane is a flat/planar plane, in others it is a warped plane. In these embodiments, the target plane is fitted to more than three points, preferably to a complete model of the organ. Using the warped plane to perform measurements might improve their accuracy since it follows the organ/muscle in 3D.

Usually, a comparison between the first model and the adjusted or improved model, both projected onto the target plane, shows that the basic plane did not capture the organ properly. In this case, the muscle's sling is for example located far out of the originally defined basic plane.

Fitting the target plane to the adjusted model may be done by assigning a point of the adjusted model to each of the characteristic points that defined the basic plane earlier, and using these points of the adjusted model to define the target plane. As is well known, a flat plane can be defined by three points. Expediently, a centre point/a point of symmetry of the adjusted model is used as one of the "new" characteristic points, e.g. the centre point of the adjusted model is assigned to the landmark that described the anorectal angle earlier. Alternatively, the points/landmarks used to define the adjusted model in the curved profile plane are used for fitting the target plane.

This method provides information which is not accessible until now and which can be a perfect basis for a further detailed analysis of the organ.

According to the disclosure, the models are each line-shaped and preferably include a set of points in 3D coordinates connected by a spline function. The spline functions can be built up using for example an appropriate MATLAB^{®} Toolbox (e.g. the Curve Fitting Toolbox). Alternatively, the models simply includes a user-defined line, or a segmented area/volume of the 3D volume.

According to one embodiment, the method comprises further the additional steps of:
i) providing a further curved profile plane which follows the improved model and extends perpendicularly to the target plane;
j) reviewing and, if necessary, adjusting the organ on the further curved profile plane, resulting in a further adjusted model of the organ;
k) fitting a further target plane to the further adjusted model;
I. optionally, projecting the further adjusted model onto the further target plane, reviewing and, if necessary, amending the further adjusted model, resulting in a further improved model of the organ.

This is essentially an iteration of the steps already performed. Thus, a further improved model of the organ can be generated.

Preferably, the basic plane and the target plane(s) are represented by rotation matrices, and respective image planes to be displayed are calculated by means of a 3D interpolation from the 3D image dataset.

The method comprises the step of performing measurements on the organ, for example in the target plane, preferably in the warped target plane, in particular measuring its length. The length of the puborectalis muscle is for example an important measurement since it provides information about the ability to let a baby through during delivery. The global longitudinal strain, hereafter called "strain" represents the amount of contraction the woman can achieve by tension of the pelvic floor. A dependency between the strain of the muscle and delivery outcome and progress is very likely.

The measurements can be made on the adjusted model. The muscle's length in 3D can be retrieved by the cumulated sum of distances of the improved model. The path starts from a first (end) point of the muscle and calculates the cumulated distance in 3D to the next point until it reaches the other end of the muscle.

At least two 3D or 4D image datasets of the same patient are provided, one while the puborectalis muscle is in the contracted state and one while it is in the relaxed state, wherein the length of the puborectalis muscle is measured in both, the contracted and the relaxed state. The ratio between these two lengths is called the strain.

According to a further advantageous embodiment, the above-described method is repeated in the same woman twice during pregnancy, in preferred embodiments once in the first half of pregnancy, and once in the second half of pregnancy, for example the first time in between the 8^{th} an 20^{th} week, for example in 12^{th} week, and the second time after the 28^{th} week, for example in 36^{th} week. In useful embodiments, there is at least 8 weeks interval between the two ultrasound sessions. The strain of the puborectalis muscle is calculated both times, and the difference between the two strains (called "delta strain") is measured. The delta strain gives an indication how much the woman's pelvic floor muscles have softened during pregnancy, and hence may be used as an important parameter in predicting the outcome of vaginal delivery.

Accordingly, the method may include providing two ultrasound image datasets of the puborectalis muscle acquired during the first half of pregnancy, one acquired in the contracted and one in the relaxed state, and two datasets acquired during the second half of pregnancy, one in the contracted and one in the relaxed state, with at least 10 weeks in between the respective ultrasound assessments, calculating the strain for each pair of datasets, and calculating the difference between the two strains.

In addition, a 3D or 4D image dataset may be provided which was acquired from a woman while the valsalva maneuver was performed. This means that the woman tries to forcefully exhale while blocking the airways.

Generally, to switch between different image datasets, preferably a slider of the graphical user interface can be used. When looking at the mid-sagittal view, the anorectal angle is visible which allows a good judgement about the current contraction or relaxation state of the muscles. Of course, the information does not have to be in several dataset which means that there can also be a single dataset which contains images acquired in the relaxed state and in the contracted state.

Preferably, during image acquisition, the women to be examined are in the supine position with an empty bladder. The transducer is preferably held to the perineum in the sagittal plane. In some embodiments, the valsalva maneuver is performed during relaxing and contracting the puborectalis muscle. The puborectalis muscle and especially the sling around the rectum moves during contraction so the two target planes (contracted and relaxed state) are tilted in different ways in the pelvic floor. The angle between those target planes, or between the basic plane and the target plane, may also be an indicator for the ability to contract the muscle and therefore for the elasticity. This angle between the planes is calculated as the angle between by the planes' normal vectors.

Preferably, the basic plane is selected for the relaxed state and the contracted state. In addition, the complete method or at least some of its steps, respectively, are performed for the relaxed and the contracted state.

According to an embodiment, the method comprises before step c, an additional step of displaying further planes parallel but shifted with respect to the basic plane. Expediently, the user can select the amount of shift with a slider on the user interface. By shifting the basic plane, the position of the basic plane with respect to the organ can be further adjusted. For example, the basic plane is shifted up to an end point of the organ, in particular an upper or lower end point. With regard to the puborectalis muscle and by keeping in mind how the muscle behaves perpendicularly in depth relative to the basic plane, the basic plane is preferably shifted to the bottom of the V- or U-shaped structure of the muscle.

According to one embodiment, defining of the organ on the various planes is performed by using a thresholding method wherein the thresholding method may be manually or semi-automatically performed. In other embodiments, defining or sketching may be performed by hand, preferably using a part of the graphical user interface, such as a mouse, touch screen or the like. The points defining the curvature of the organ can be defined in the 3D image dataset by setting appropriate landmarks. Expediently, the user interface is appropriately adapted to enable different views onto the organ or body region to be examined.

The models are line shaped and preferably include a set of points on the respective plane, preferably connected by a spline function. These points are preferably identified by setting appropriate landmarks on the displayed image planes. According to one embodiment, first a set of basic landmarks is set on the basic plane which defines a first rough outline of the organ. Preferably, additional landmarks are set to outline the muscle in 2D more precisely. These landmarks provide more accurate information about the length and shape of the strand-shaped muscle. Advantageously, the spline function follows automatically the set landmarks. These new landmarks are preferably transferred to the data volume in the same way as the basic landmarks using the rotation matrix (or matrices).

With regard to the puborectalis muscle, preferably a set of landmarks/points comprises three basic landmarks wherein a first basic landmark is positioned basically in the centre of the strand-shaped muscle, and the other basic landmarks define the start and end points of the strand. In particular, the first landmarks is preferably at the sling or the centre of the sling of the puborectalis muscle, wherein the other two landmarks define the positions where the puborectalis muscle is attached to the pubic bone. Each landmark may be back-projected from the respective 2D plane into the data volume (X, Y, Z) by multiplication with the appropriate rotation matrix.

In an alternative embodiment, the image dataset is a 4D dataset, i.e. a time series of 3D image datasets acquired from the pelvic floor of a woman, preferably during a contraction of the puborectalis muscle. Such contraction may last 1 to 3 seconds, and the image datasets are preferably acquired at a rate of 3 to 30 volumes (=3D datasets) per second, more preferred at around 6 to 15 volumes per second.

On the first 3D image of this time series, the puborectalis muscle is defined/segmented by the method as described above. Most preferred, not only a line-shaped model is defined, but the muscle is segmented or outlined at its borders, so that at least some voxels which belong to the puborectalis muscle, or a volume representing the puborectalis muscle, are defined.

According to a preferred embodiment, the method then uses speckle tracking within the muscle tissue to track the muscle during its contraction movement through the time series of the 4D image dataset. In order words, the muscle tissue is tracked from one volume (also called "frame") to the next, so that a model or definition of the muscle exists on each 3D image belonging to the time series. "Speckle" in ultrasound images is caused by incidental echo scatter. Although it is a kind of noise, it moves with the tissue and can therefore be used to track a certain structure over several images. By this technique, it will be possible to calculate also regional strain within the puborectalis muscle, for example the strain on the right half and the strain on the left half, to thereby refine the analysis.

The segmentation of the puborectalis muscle on a 3D image dataset may be done by using the average grey value intensity. For example, the landmarks are set by the user as before on the basic plane and/or the curved profile plane, and the grey value is taken from these points/landmarks. In a next step, the computer or calculating unit automatically segments the muscle using a threshold close to the average of the landmarks' grey values. Thereby, the complete volume of the puborectalis muscle is determined.

In an alternative embodiment, the method for analysing the puborectalis muscle is performed mostly automatically, but giving the user the possibility for review. According to a preferred embodiment, the method is as follows:
a. providing at least one 3D or 4D image dataset which includes the organ;
b. automatically selecting three landmarks defining a basic plane through the 3D image dataset, which at least partially intersects the organ, wherein the landmarks include the points (B1) where the muscle is attached to the bone, and one further landmark (S1);
c. optionally displaying the basic plane for review by a user, who has the possibility of adjusting the landmarks, and defining the organ on the basic plane, resulting in a model of the organ;
d. automatically adjusting the position of the landmarks, in particular of the further landmark, and displaying a target plane which is spanned by the adjusted landmarks;
e. displaying the target plane with the model of the organ and optionally adjusting the organ on the target plane, resulting in an improved model of the organ.

Accordingly, the curved profile plane is not necessarily used, but rather the position of the landmarks, in particular the three basic landmarks mentioned above, namely the points where the muscle is attached to the bone, and one sling landmark, are automatically adjusted, starting from the basic plane. Since the landmarks where the muscle is attached to the bone are more likely to be correct at fest, especially the sling landmark is adjusted automatically. Usually, at the end, the target plane is displayed, with the model of the organ projected onto it, giving the user the possibility to review. Optionally, he may adjust the model or the landmarks spanning the model, resulting in an improved model of the organ. Optionally, a curved profile plane may also be displayed for review by the user.

The automatic adjustment of the position of the landmarks may be trained, using machine learning methods, for example using deep artificial neural networks. In other words, many examples of user-selected landmarks and models are fed to the computer so that he can learn where to look for the correct landmarks.

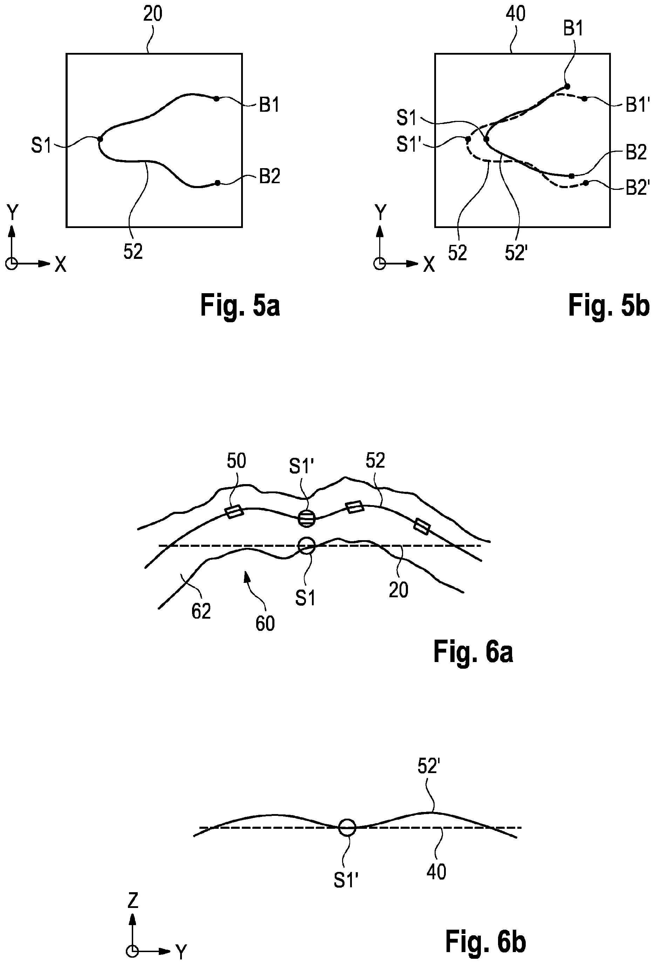

The planes such as shown in Fig. 5a/5b and/or 6a/6b may be shown to the user for review and possible correction of the model.

The disclosure also includes a computer program comprising software which causes a computer to perform the method according to the invention if the computer program is performed on the computer.

The disclosure is also directed to a device for analysing a non-planar organ in the human or animal body, in particular a strand-shaped organ, comprising
- a data storage for digitally storing a 3D or 4D image dataset of the organ or the respective body region;
- a user interface which comprises a display unit which is adapted to display planes through the 3D image dataset;
- a calculating unit which is adapted to perform the method according to the invention;
- a user input device allowing a user to select points on a displayed plane.

The device can be any kind of computer, e.g. a workstation, PC or even a mobile device, laptop, tablet etc. The user interface preferably comprises a part for enabling the user to select landmarks on a screen, e.g. a touch screen, mouse etc.

The features of the method according to the invention apply also to the computer program and the device according to the invention and vice versa. Further feature and advantages are presented with regard to the following figures. Different embodiments shown are explicitly allowed to be combined.
- Figures 1a-c: show an embodiment of the method of defining a model of an organ on a basic plane using a graphical user interface;
- Figure 2a: shows a basic plane which is the basis for a curved profile plane;
- Figure 2b: shows a view onto the curved profile plane;
- Figure 3: shows a model of the organ;
- Figure 4: shows projecting of landmarks/points to a target plane;
- Figures 5a, 5b: show a model in a basic plane and the projected model in a target plane;
- Figures 6a, 6b: show models in the curved profile plane before and after adjustment.

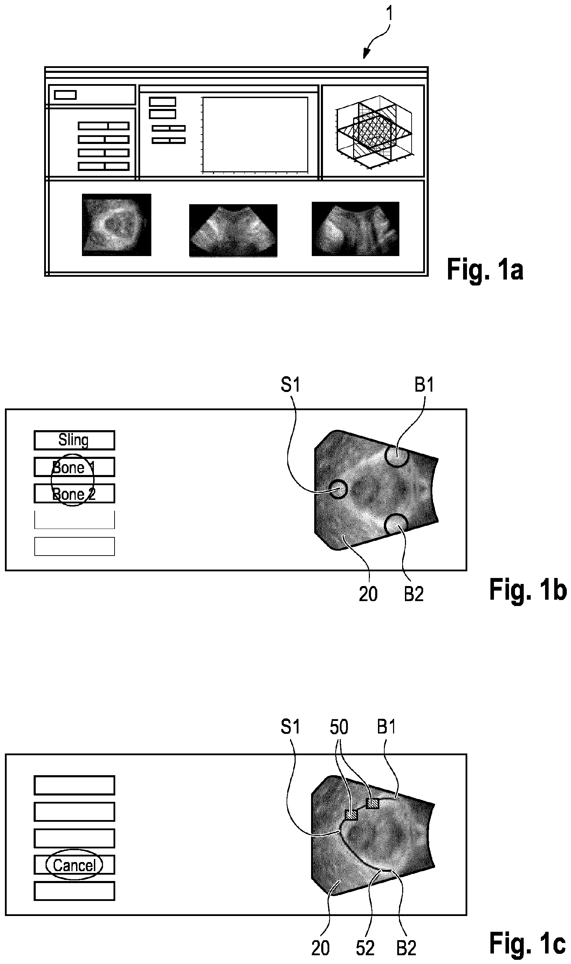

**Fig. 1a** shows a graphical user interface 1 after loading of a 3D image dataset, as shown on the screen of a computer or other calculating device. Different slices/views of the body region to be examined are shown in three display areas in the bottom part of the graphical user interface 1. Expediently, various sliders, buttons and display areas are provided to perform the method of analysing the non-planar organ. For example, to start with, three orthogonal slices through a 3D image dataset are displayed in the three display areas, and the sliders can be used to shift the slices through the volume, i.e. to navigate.

This can be used to determine a basic plane. In the case the strand-shaped organ is the puborectalis muscle, for example a landmark for the pubic symphysis is selected in the 3D image dataset, or rather on a slice through it (not shown). This point is for example visible in the XY plane where a bone structure appears anteriorly in reality. The bones have to be as close together as possible but still clearly separable. Alternatively, the pubic symphysis landmark can also be identified, e. g. in the coronal plane. When the pubic symphysis is marked, the posterior border of the levator hiatus is the subject to find. The anorectal angle is visible in the mid-sagittal view where also the contraction state was determined. In reality, the structure posterior to the anorectal angle is a cross-section of the puborectalis muscle. On the border of this structure as close to the symphysis landmark as possible, the puborectalis landmark is set. With these two points the basic plane or, in this case, the plane of minimal dimensions is defined as a tilted axial plane. Preferably the basic plane/the plane of minimal dimensions can be tilted around the (basic) axis which is defined by the respective landmarks. Issues during the ultrasound examination that made this plane unsymmetrical can be counteracted that way. In addition, the basic plane can be shifted parallel up- or downwards to get an idea of the muscle behaviour in depth perpendicular to the basic plane for better landmark selection in the following steps. Preferably, using for example an appropriate slider of a user interface, the basic plane can be adjusted.

**Fig. 1b** shows such a view onto a basic plane 20 and the setting of basic landmarks B1, B2 and S1. The puborectalis muscle is faintly visible as a light, slingshaped area. The centre basic landmark or sling landmark S1 is preferably placed on the bottom of the V- or U-shaped structure of the muscle. The basic plane 20 with its settings for the angle and the parallel shift is preferably defined by a rotation matrix which is also needed in the following steps to back-project other landmarks. When the basic plane 20 is found, the organ is sketched on it using basic landmarks 51 which define a first rough outline of the muscle/organ to be examined. With regard to the puborectalis muscle, the first basic landmark is preferably the sling S1 of the puborectalis muscle, as already mentioned, wherein the other two basic landmarks B1 and B2 define the positions where the puborectalis muscle is attached to the pubic bone. Further landmarks 50 are used to specify the outline of the organ in more detail. Based on this outline or these landmarks, a model of the organ can be generated, e.g. by fitting a spline function through the landmarks. The three landmarks S1, B1, B2 also define the orientation of the basic plane.

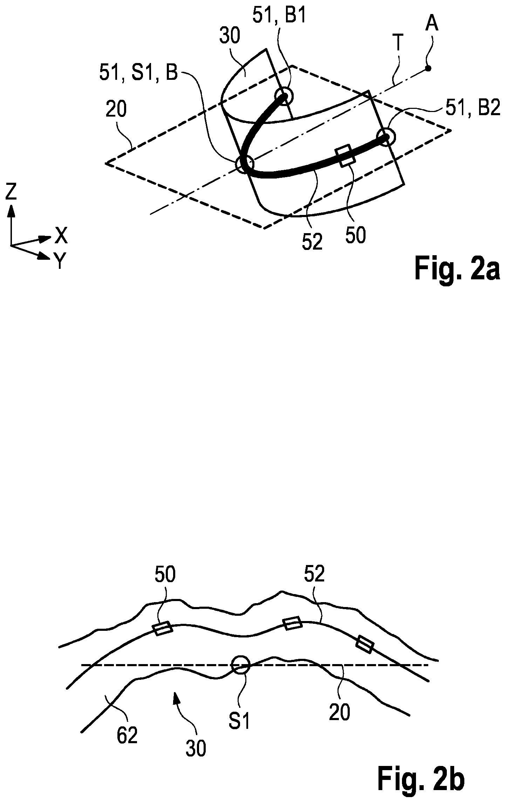

This model 52 is shown in **Fig. 1c****.** Further landmarks 50 can be set to define/specify the model 52 of the organ in more detail. The model 52 is used to define a curved profile plane which is shown in Fig. 2a.

**Fig. 2a** shows a perspective view of the basic plane 20. The basic plane 20 was selected through the 3D image dataset (preferably generated using a 3D or 4D ultrasound system) of the pelvic floor area, wherein the basic plane 20 at least partially intersects the puborectalis muscle. A landmark A for the pubic symphysis was set in the 3D image dataset or a slice through it. Reference numeral B identifies the anorectal angle. With these two points the basic plane 20 or, in this case, the plane of minimal dimensions is defined as a tilted axial plane. The (basic) axis T is shown which is defined by the points/landmarks A and B. The model 52 was sketched in the basic plane 20 and is now used to define a curved profile plane 30 which extends perpendicular to the basic plane 20. The curved profile plane 30 is now used to examine how the organ behaves in the depth direction, i.e. perpendicular to the basic plane.

**Fig. 2b** shows a view onto the curved profile plane 30, perpendicularly to the basic plane 20. The organ is identified by reference numeral 62. One can see that the puborectalis muscle 62 is not perfectly lying in the basic plane 20, but meanders above and below it. The user then sketches the shape of the organ by selecting further landmarks 50, and the computer fits a model 53 through the landmarks 50. Thus, landmarks 50 are used to define an adjusted model 53.

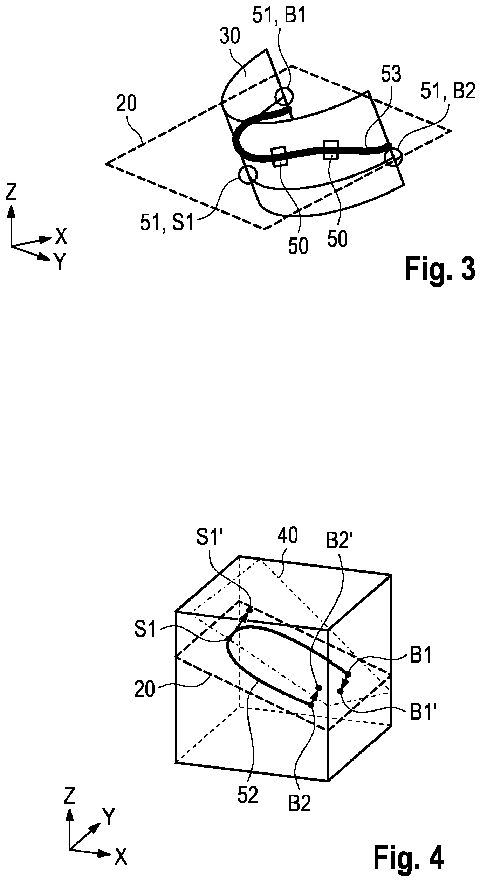

**Fig. 3** shows this adjusted model 53 in a perspective view. A comparison between the model 53 and the basic plane 20 shows that the basic plane 20 did not capture the organ properly. In this case the muscle's sling is for example located far out of the originally defined basic plane 20. Preferably, a target plane 40 is generated to replace the basic plane 20. This is preferably done by using three basic landmarks of the adjusted model, namely the adjusted sling landmark S1', as well as the new endpoints of the puborectalis muscle, B1' and B2', see also Fig. 4. The plane which is spanned by these three points can be used as target plane 40.

**Fig. 4** shows a basic plane 20 comprising basic landmarks B1, B2 and S1 and a target plane 40, spanned by adjusted basic landmarks B1', B2' and S1'. The arrows indicate how the landmarks have moved. As one can see, also the orientation angle of the target plane 40 is different to that of the basic plane 20.

**Fig. 5a** shows a top view (XY plane) of a spline function 52 in a basic plane 20. The spline function/model 52 defines the curvature of the curved profile plane. Of course, in this view, the curved profile plane is also just visible as line.

**Fig. 5b** shows a top view (XY plane) of a target plane 40. The projection of former model 52 of Fig. 4a onto the target plane is visible as a dotted line. The adjusted or improved model 52' is shown, as projected onto the target plane 40. It can be seen that the deviations between the lines / models 52 and 52' are big and therefore, the position/location of the basic plane 20 was not very exact.

**Fig. 6a** shows schematically image data 60 wherein an image of an organ is identified by reference numeral 62. Reference numeral 20 indicates a basic plane 20. It's a view onto a curved profile plane in order to outline the organ in depth. The person skilled in the art knows that organ structures in ultrasound images are often only dimly displayed or represented. It was tried to follow the shape of the organ using the model 52. In other words landmarks 50 were identified and the landmarks 50 were used to generate the model/spline function 52. Its position relative to the spline function 52 is not very good, cf. to the position of the basic landmarks S1 and S1'. The basic landmark S1' does not lie within the basic plane 20.

**Fig. 6b** shows a target plane 40. The model 52' is positioned much better with regard to the appropriate plane. The basic landmark S1' lies perfectly on the target plane 40.

### Reference numerals

- 1: graphical user interface
- 20: basic plane
- 30: curved profile plane
- 40: target plane
- 50: points, landmarks
- 51: basic landmarks
- 52: spline-function, 2D definition
- 52': projected/adjusted 2D definition
- 53: spline function, 3D definition
- 60: image data
- 62: organ image
- A: landmark, pubic symphysis
- B: landmark, puborectalis muscle
- S1: center basic landmark, sling landmark
- B1: first basic end landmark, bone landmark 1
- B2: second basic end landmark, bone landmark 2
- S1', B1', B2': adjusted basic landmarks
- X, Y, Z: coordinates
- T: (basic) axis

## Claims

1. Method for analyzing a non-planar organ (62) in the human or animal body, on a computer or other computing device, the method comprising the steps:
a. providing at least one 3D or 4D image dataset (60) which includes the organ (62);
b. selecting a basic plane (20) through the 3D image dataset (60) which at least partially intersects the organ (62);
c. displaying the basic plane (20) and defining the organ (62) on the basic plane (20) by sketching the shape of the organ (62), resulting in a line-shaped model (52) of the organ (62);
d. providing a curved profile plane (30) which follows the curvature of the line of the line-shaped model (52) and extends perpendicular to the basic plane (20);
e. displaying the curved profile plane (30) and defining the organ (62) on the curved profile plane (30) by sketching the shape of the organ (62), resulting in a line-shaped adjusted model (53) of the organ (62);
wherein the organ (62) is the puborectalis muscle, and
the method further comprising the step of performing length measurements on the adjusted model (53) of the organ (62),
wherein the method is performed on two datasets, one acquired while the puborectalis muscle is in a relaxed state and one where the puborectalis muscle is in a contracted state, and the ratio between the lengths of the organ models (53) in the relaxed and contracted states is calculated, to obtain a measure of the strain.

2. Method according to claim 1,
wherein the models (52, 52', 53) are spanned by a set of points (50), which are sketched by a user on the respective plane during the defining of the organ (62), and are connected by a spline-function (52, 52', 53).

3. Method according to any one of the preceding claims, comprising the additional steps:
f. fitting a target plane (40) to the adjusted model (53) or a few landmarks (50) defining the adjusted model (53); and
g. displaying the target plane (40) and projecting the adjusted model (53) onto the target plane (40) for review by a user;
h. optionally, further amending the model (53) to fit it to the organ (62) on the target plane (40), resulting in an improved model (52') of the organ (62).

4. Method according to claim 3, comprising the additional steps of:
i. providing a further curved profile plane (30) which follows the improved model (52');
j. defining the organ (62) on the further curved profile plane (30), resulting in a further adjusted model (53) of the organ (62);
k. fitting a further target plane (40) to the further adjusted model (53);
l. projecting the further adjusted model (53) onto the further target plane (40), reviewing and, if necessary, amending the further adjusted model (53), resulting in a further improved model (52') of the organ (62).

5. Method according to any one of the preceding claims, wherein the target plane (40) or the further target plane (40) is a warped plane.

6. Method according to any one of the preceding claims, comprising the step of performing measurements on a planar target plane (40), in particular measuring the angle of the planar target plane (40).

7. Method according to any one of the preceding claims, wherein the defining of the organ (62) on the various planes is performed by using a thresholding method, wherein the thresholding method may be semi-automatically performed.

8. Method according to any of the preceding claims, wherein the dataset is a 4D dataset comprising 3-30 volumes per second, acquired during contraction of the puborectalis muscle, and wherein speckle tracking of the tissue of the puborectalis muscle is performed to thereby track the muscle tissue from one volume to the next.

9. Method according to any one of the preceding claims, which comprises
- providing two ultrasound image datasets (60) of the puborectalis muscle acquired during the first half of pregnancy, one acquired in the contracted and one in the relaxed state,
- providing two ultrasound image datasets (60) of the puborectalis muscle of the same woman acquired during the second half of pregnancy, one in the contracted and one in the relaxed state,
- calculating the strain for each pair of datasets;
- and calculating the difference between the two strains.

10. A computer program, comprising software which causes a computer to perform the method according to any of claims 1 to 9 if the computer program is run on the computer.

11. A device for analysing a non-planar organ (62) in the human or animal body, in particular a strand-shaped organ (62) such as the puborectalis muscle, comprising
- a data storage for digitally storing a 3D or 4D image dataset (60) of the organ (62) or the respective body region;
- a user interface (1) which comprises a display unit which is adapted to display planes through the 3D image dataset (60);
- a calculating unit which is adapted to perform the method according to any one of claims 1 to 9;
- a user input device allowing a user to select points on a displayed plane.

## Patentansprüche

1. Verfahren zum Analysieren eines unebenen Organs (62) im menschlichen oder tierischen Körper auf einem Computer oder einer anderen Rechenvorrichtung, wobei das Verfahren die folgenden Schritte umfasst:
a. Bereitstellen mindestens eines 3D- oder 4D-Bilddatensatzes (60), der das Organ (62) beinhaltet;
b. Auswählen einer Basisebene (20) durch den 3D-Bilddatensatz (60), die das Organ (62) mindestens teilweise schneidet;
c. Anzeigen der Basisebene (20) und Definieren des Organs (62) in der Basisebene (20) durch Skizzieren der Form des Organs (62), was zu einem linienförmigen Modell (52) des Organs (62) führt;
d. Bereitstellen einer gekrümmten Profilebene (30), die der Krümmung der Linie des linienförmigen Modells (52) folgt und sich senkrecht zur Basisebene (20) erstreckt;
e. Anzeigen der gekrümmten Profilebene (30) und Definieren des Organs (62) in der gekrümmten Profilebene (30) durch Skizzieren der Form des Organs (62), was zu einem linienförmigen angepassten Modell (53) des Organs (62) führt;
wobei das Organ (62) der Musculus puborectalis ist, und
wobei das Verfahren weiter den Schritt des Durchführens von Längenmessungen an dem angepassten Modell (53) des Organs (62) umfasst,
wobei das Verfahren an zwei Datensätzen durchgeführt wird, einem, der erfasst wird, während sich der Musculus puborectalis in einem entspannten Zustand befindet, und einem, bei dem sich der Musculus puborectalis in einem angespannten Zustand befindet, und das Verhältnis zwischen den Längen der Organmodelle (53) im entspannten und im angespannten Zustand berechnet wird, um ein Maß der Dehnung zu erhalten.

2. Verfahren nach Anspruch 1, wobei die Modelle (52, 52', 53) durch eine Punktmenge (50) aufgespannt werden, die von einem Benutzer während des Definierens des Organs (62) in der jeweiligen Ebene skizziert werden, und durch eine Spline-Funktion (52, 52', 53) verbunden sind.

3. Verfahren nach einem der vorstehenden Ansprüche, das die folgenden zusätzlichen Schritte umfasst:
f. Anpassen einer Zielebene (40) an das angepasste Modell (53) oder einiger Orientierungspunkte (50), die das angepasste Modell (53) definieren; und
g. Anzeigen der Zielebene (40) und Projizieren des angepassten Modells (53) auf die Zielebene (40) zur Überprüfung durch einen Benutzer;
h. gegebenenfalls weiteres Ändern des Modells (53), um es in der Zielebene (40) an das Organ (62) anzupassen, was zu einem verbesserten Modell (52') des Organs (62) führt.

4. Verfahren nach Anspruch 3, das die folgenden zusätzlichen Schritte umfasst von:
i. Bereitstellen einer weiteren gekrümmten Profilebene (30), die dem verbesserten Modell (52') folgt;
j. Definieren des Organs (62) in der weiteren gekrümmten Profilebene (30), was zu einem weiter angepassten Modell (53) des Organs (62) führt;
k. Anpassen einer weiteren Zielebene (40) an das weiter angepasste Modell (53);
l. Projizieren des weiter angepassten Modells (53) auf die weitere Zielebene (40), Überprüfen und, soweit erforderlich, Ändern des weiter angepassten Modells (53), was zu einem weiter verbesserten Modell (52') des Organs (62) führt.

5. Verfahren nach einem der vorstehenden Ansprüche, wobei die Zielebene (40) oder die weitere Zielebene (40) eine verzerrte Ebene ist.

6. Verfahren nach einem der vorstehenden Ansprüche, das den Schritt des Durchführens von Messungen in einer ebenen Zielebene (40), insbesondere des Messens des Winkels der ebenen Zielebene (40), umfasst.

7. Verfahren nach einem der vorstehenden Ansprüche, wobei das Definieren des Organs (62) in den verschiedenen Ebenen unter Verwendung eines Schwellenwertverfahrens durchgeführt wird, wobei das Schwellenwertverfahren halbautomatisch durchgeführt werden kann.

8. Verfahren nach einem der vorstehenden Ansprüche, wobei der Datensatz ein 4D-Datensatz ist, der 3-30 Volumina pro Sekunde umfasst, die während Anspannung des Musculus puborectalis erfasst werden, und wobei Speckle-Tracking des Gewebes des Musculus puborectalis durchgeführt wird, um dadurch das Muskelgewebe von einem Volumen zum nächsten zu verfolgen.

9. Verfahren nach einem der vorstehenden Ansprüche, das umfasst
- Bereitstellen von zwei Ultraschall-Bilddatensätzen (60) des Musculus puborectalis, die während der ersten Schwangerschaftshälfte erfasst werden, wobei einer im angespannten und einer im entspannten Zustand erfasst wird,
- Bereitstellen von zwei Ultraschall-Bilddatensätzen (60) des Musculus puborectalis derselben Frau, die während der zweiten Schwangerschaftshälfte, einer im angespannten und einer im entspannten Zustand, erfasst werden,
- Berechnen der Dehnung für jedes Datensatzpaar;
- und Berechnen der Differenz zwischen den zwei Dehnungen.

10. Computerprogramm, das Software umfasst, die einen Computer dazu bringt, das Verfahren nach einem der Ansprüche 1 bis 9 durchzuführen, wenn das Computerprogramm auf dem Computer ausgeführt wird.

11. Vorrichtung zum Analysieren eines unebenen Organs (62) im menschlichen oder tierischen Körper, insbesondere eines strangförmigen Organs (62) wie etwa des Musculus puborectalis, umfassend
- einen Datenspeicher zum digitalen Speichern eines 3D- oder 4D-Bilddatensatzes (60) des Organs (62) oder der jeweiligen Körperregion;
- eine Benutzerschnittstelle (1), die eine Anzeigeeinheit umfasst, die geeignet ist, Ebenen durch den 3D-Bilddatensatz (60) anzuzeigen;
- eine Recheneinheit, die geeignet ist, das Verfahren nach einem der Ansprüche 1 bis 9 durchzuführen;
- eine Benutzereingabevorrichtung, die es einem Benutzer ermöglicht, Punkte in einer angezeigten Ebene auszuwählen.

## Revendications

1. Procédé d'analyse d'un organe non plan (62) dans le corps humain ou animal, sur un ordinateur ou autre dispositif informatique, le procédé comprenant les étapes de :
a. fourniture d'au moins un ensemble de données d'image 3D ou 4D (60) qui inclut l'organe (62) ;
b. sélection d'un plan de base (20) à travers l'ensemble de données d'image 3D (60) qui coupe au moins partiellement l'organe (62) ;
c. affichage du plan de base (20) et définition de l'organe (62) sur le plan de base (20) en traçant la forme de l'organe (62), ce qui donne un modèle en forme de ligne (52) de l'organe (62) ;
d. fourniture d'un plan de profil incurvé (30) qui suit la courbure de la ligne du modèle en forme de ligne (52) et s'étend perpendiculairement au plan de base (20) ;
e. affichage du plan de profil incurvé (30) et définition de l'organe (62) sur le plan de profil incurvé (30) en traçant la forme de l'organe (62), ce qui donne un modèle ajusté en forme de ligne (53) de l'organe (62) ;
dans lequel l'organe (62) est le muscle pubo-rectal, et
le procédé comprenant en outre l'étape de réalisation de mesures de longueur sur le modèle ajusté (53) de l'organe (62),
dans lequel le procédé est exécuté sur deux ensembles de données, l'un acquis lorsque le muscle pubo-rectal est dans un état détendu et l'autre dans lequel le muscle pubo-rectal est dans un état contracté, et le rapport entre les longueurs des modèles d'organes (53) dans les états détendu et contracté est calculé pour obtenir une mesure de la déformation.

2. Procédé selon la revendication 1, dans lequel les modèles (52, 52', 53) sont couverts d'un ensemble de points (50) qui sont tracés par un utilisateur sur le plan respectif lors de la définition de l'organe (62), et sont reliés par une fonction spline (52, 52', 53).

3. Procédé selon l'une quelconque des revendications précédentes, comprenant les étapes supplémentaires de :
f. adaptation d'un plan cible (40) au modèle ajusté (53) ou quelques repères (50) définissant le modèle ajusté (53) ; et
g. affichage du plan cible (40) et projection du modèle ajusté (53) sur le plan cible (40) pour examen par un utilisateur ;
h. facultativement, modification supplémentaire du modèle (53) pour l'adapter à l'organe (62) sur le plan cible (40), ce qui aboutit à un modèle amélioré (52') de l'organe (62).

4. Procédé selon la revendication 3, comprenant les étapes supplémentaires de :
i. fourniture d'un autre plan de profil incurvé (30) qui suit le modèle amélioré (52') ;
j. définition de l'organe (62) sur le plan de profil davantage incurvé (30), ce qui donne lieu à un modèle davantage ajusté (53) de l'organe (62) ;
k. adaptation d'un autre plan cible (40) au modèle davantage ajusté (53) ;
l. projection du modèle davantage ajusté (53) sur l'autre plan cible (40), examen et, si nécessaire, modification du modèle davantage ajusté (53), ce qui aboutit à un modèle davantage amélioré (52') de l'organe (62).

5. Procédé selon l'une quelconque des revendications précédentes, dans lequel le plan cible (40) ou l'autre plan cible (40) est un plan déformé.

6. Procédé selon l'une quelconque des revendications précédentes, comprenant l'étape de réalisation de mesures sur un plan cible plan (40), en particulier la mesure de l'angle du plan cible plan (40).

7. Procédé selon l'une quelconque des revendications précédentes, dans lequel la définition de l'organe (62) sur les différents plans est réalisée en utilisant un procédé de seuillage, dans lequel le procédé de seuillage peut être réalisé de manière semi-automatique.

8. Procédé selon l'une quelconque des revendications précédentes, dans lequel l'ensemble de données est un ensemble de données 4D comprenant 3-30 volumes par seconde, acquis pendant la contraction du muscle pubo-rectal, et dans lequel un suivi de granularité du tissu du muscle pubo-rectal est effectué pour ainsi suivre le tissu du muscle d'un volume à l'autre.

9. Procédé selon l'une quelconque des revendications précédentes, qui comprend
- la fourniture de deux ensembles de données d'images échographiques (60) du muscle pubo-rectal acquis au cours de la première moitié de la grossesse, un acquis à l'état contracté et un à l'état détendu,
- la fourniture de deux ensembles de données d'images échographiques (60) du muscle pubo-rectal de la même femme, acquis au cours de la seconde moitié de la grossesse, un à l'état contracté et un à l'état détendu,
- le calcul de la déformation pour chaque paire d'ensembles de données ;
- et le calcul de la différence entre les deux déformations.

10. Programme informatique, comprenant un logiciel qui amène un ordinateur à exécuter le procédé selon l'une quelconque des revendications 1 à 9 si le programme informatique est exécuté sur l'ordinateur.

11. Dispositif d'analyse d'un organe non plan (62) dans le corps humain ou animal, en particulier un organe en forme de brin (62) tel que le muscle pubo-rectal, comprenant
- un stockage de données pour stocker numériquement un ensemble de données d'image 3D ou 4D (60) de l'organe (62) ou de la région corporelle respective ;
- une interface utilisateur (1) qui comprend une unité d'affichage qui est conçue pour afficher des plans par l'intermédiaire de l'ensemble de données d'image 3D (60) ;
- une unité de calcul qui est conçue pour exécuter le procédé selon l'une quelconque des revendications 1 à 9 ;
- un périphérique de saisie utilisateur permettant à un utilisateur de sélectionner des points sur un plan affiché.
